(19) Europäisches Patentamt
European Patent Office
Office européen des brevets

(11) Veröffentlichungsnummer: **0 112 502**
**B1**

(12) # EUROPÄISCHE PATENTSCHRIFT

(45) Veröffentlichungstag der Patentschrift: **19.06.85**

(21) Anmeldenummer: **83111777.5**

(22) Anmeldetag: **24.11.83**

(51) Int. Cl.⁴: **C 07 C 69/732,** C 07 C 69/16, C 07 C 67/343, C 07 C 67/293, C 07 D 319/06, C 07 C 175/00

(54) **Omega,omega-Diacyloxy-2,6-dimethyl-octatriensäureester und -aldehyde, Verfahren zu ihrer Herstellung und Verwendung zur Synthese von Terpenverbindungen.**

(30) Priorität: **30.11.82 DE 3244272**

(43) Veröffentlichungstag der Anmeldung: **04.07.84 Patentblatt 84/27**

(45) Bekanntmachung des Hinweises auf die Patenterteilung: **19.06.85 Patentblatt 85/25**

(84) Benannte Vertragsstaaten: **CH DE FR GB LI NL**

(56) Entgegenhaltungen:
**FR - A - 2 300 760**

**HELVETICA CHEMICA ACTA, Band 49, 1966, Seiten 369-390, Basel, CH. U. SCHWIETER et al.: "Synthesen in der Carotinoid-Reihe. Neue Synthesen von Apocarotinoiden"**

(73) Patentinhaber: **BASF Aktiengesellschaft, Carl-Bosch-Strasse 38, D-6700 Ludwigshafen (DE)**

(72) Erfinder: **Schmieder, Klaus, Dr., Sonnenstrasse 12 b, D-6710 Frankenthal (DE)**
Erfinder: **Paust, Joachim, Dr., Ringstrasse 3, D-6708 Neuhofen (DE)**
Erfinder: **Fischer, Rolf, Dr., Bergstrasse 98, D-6900 Heidelberg (DE)**
Erfinder: **Weitz, Hans-Martin, Dr., Auf dem Koeppel 40, D-6702 Bad Duerkheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 112 502 B1

BUNDESDRUCKEREI BERLIN

**Beschreibung**

Die Erfindung betrifft neue bifunktionelle, unsymmetrische $C_{10}$-Bausteine für Terpensynthesen, die an einem Kettenende eine Formyl- oder Alkoxycarbonylgruppe und am anderen Kettenende 2 geminale Acyloxygruppen tragen sowie ein Verfahren zu deren Herstellung aus leicht zugänglichen technischen $C_5$-Bausteinen und die Verwendung zur Synthese von Terpenverbindungen durch übliche Carbonylolefinierung unter an sich bekannten Bedingungen.

Aus Helv. Chim. Acta 49, 369 (1966), bzw. der US-Patentschrift 3 989 758 sind $C_{10}$-Bausteine des Typs, wie sie aus Verbindungen der Formel I nach Hydrolyse der Acylalfunktion entstehen, bekannt. Auch ihre Verwendung für die Synthesen von $\beta$-Apo-8′-carotinal und $\beta$-Apo-8′-carotinsäureester ist dort beschrieben. Die Herstellung dieser $C_{10}$-Bausteine ist bisher jedoch nur nach komplizierten vielstufigen Synthesen möglich. So wird 2,6-Dimethyl-8-oxo-octa-2,4,6-trien-1-säureethylester nach Helv. Chim. Acta l. c. in einer sechsstufigen Synthese mit einer Gesamtausbeute unter 35% hergestellt. Für die Synthese von 8,8-Dimethoxi-3,7-dimethyl-octa-2,4,6-trien-1-al benötigt man nach Helv. Chim. Acta l. c. und J. Chem. Soc. 1965, 2019, sogar 8 Stufen und erhält eine Ausbeute von unter 10%.

Es bestand daher die Aufgabe für die Synthese von $\beta$-Apo-8′-carotinal, $\beta$-Apo-8′-carotinsäureester und für andere Synthesen in der Terpenchemie $C_{10}$-Bausteine vorzuschlagen, die sich für diesen Zweck ebenso eignen wie die in der genannten Vorliteratur beschriebenen $C_{10}$-Aldehyde, die aber leichter synthetisierbar sind.

Es wurde nun überraschenderweise gefunden, daß diese Aufgabe mit Verbindungen der Formel I

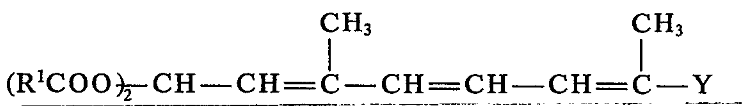

in der Y für den Rest $-COOR^1$, eine Formylgruppe oder eine acetalisierte Formylgruppe steht, wobei $R^1$ einen niedermolekularen Alkylrest bedeutet, gelöst wird.

In dieser Formel bedeutet $R^1$ vorzugsweise einen geradkettigen oder verzweigten Alkylrest mit 1 bis 5 Kohlenstoffatomen und im Falle von Y einer acetalisierten Formylgruppe den Rest der Formel

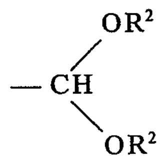

in der $R^2$ einen geradkettigen oder verzweigten Alkylrest mit 1 bis 5 Kohlenstoffatomen oder eine Alkylen- oder Alkenylengruppe mit 2 bis 4 Kohlenstoffatomen in der Kette bedeutet.

Die neuen Verbindungen der Formel I werden z. B. erhalten, wenn man Verbindungen der Formel II

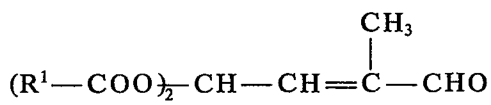

mit Triarylphosphoniumsalzen der Formel III

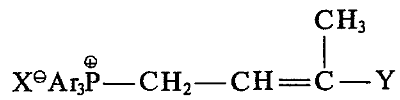

in der X Chlor oder Brom bedeutet und $R^1$ und Y die oben angegebenen Bedeutungen haben in Gegenwart von Alkylepoxiden oder unter nichtprotonischen basischen Bedingungen umsetzt. Die Wittig-Reaktion in Gegenwart von Alkylepoxiden ist als Buddrus-Variante an sich in der Literatur bekannt, vgl. DP-1 768 680 bzw. Chem. Ber. 107, 2050 (1974); eine Anwendung in Verbindung mit den hochempfindlichen Acylalen ist jedoch bisher nicht beschrieben. Auf diese Literaturstelle wird bezüglich der Reaktionsbedingungen Bezug genommen.

Zweckmäßig führt man die Umsetzung im einzelnen so durch, daß man äquivalente Mengen der Verbindung II unter der Verbindung III in 1—10 Äquivalenten Alkylepoxid bei 0—60°C rührt. Als Lösungsmittel dient entweder überschüssiges Alkylepoxid oder beliebige andere unter den Reaktionsbedingungen inerte Lösungsmittel wie Methylenchlorid, Dimethylformamid, Methanol oder Tetrahydrofuran. Die Trennung des Wertproduktes von ebenfalls gebildeten Triphenylphosphinoxid kann entweder durch Waschen mit wäßrigem Dimethylformamid bzw. wäßrigem Methanol oder durch Kristallisieren entweder von Triphenylphosphinoxid oder vom Wertprodukt erfolgen.

Alternativ kann die Herstellung der Verbindungen vom Typ I durch Wittig-Reaktion von II mit III unter nichtprotonischen basischen Bedingungen (z. B. Butyllithium/Tetrahydrofuran in an sich bekannter Weise — vgl. A. Maercker, Org. Reactions, Vol. 14, S. 217—490) erfolgen. In disem Fall wird ein Phosphoniumsalz des Typs III z. B. in Tetrahydrofuran suspendiert, unter Kühlung in eine Schutzgasatmosphäre mit Butyllithium und dann mit einem Aldehyd II versetzt. Nach beendeter Reaktion wird überschüssiges Butyllithium mit Methanol/Wasser zerstört und das Reaktionsgemisch in üblicher Weise aufgearbeitet.

Eine andere Herstellungsmöglichkeit beseht in der Umsetzung des Phosphonats IV der Formel

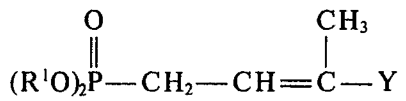

in der $R^1$ und Y die oben angegebenen Bedeutungen haben mit Acylalen der Formel II unter den Bedingungen der Wittig-Horner-Reaktion, wie sie in Chem. Rev. 74, 87 (1974), beschrieben ist. Auf die dort angegebenen Reaktionsbedingungen wird Bezug genommen.

Beispielsweise führt man diese Umsetzung im einzelnen so aus, daß man aus IV das Phosphonat-Anion bei Raumtemperatur bis 50°C mit Natriumhydrid in Tetrahydrofuran bildet und dann die Carbonylkomponente II zutropft und nachrührt bis die Umsetzung vollständig ist.

Die so erhaltenen neuen Verbindungen der Formel I können entweder ohne Isolierung oder nach Isolierung durch Extraktion oder Kristallisation weiterverarbeitet werden, wobei für Y = $COOR^1$ und Y =

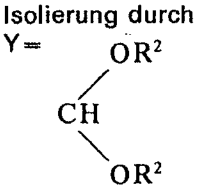

übliche basische Bedingung einer Wittig-Reaktion in protonischen Medien (z. B. NaOEt/EtOH) für eine selektive Hydrolyse der Acylalfunktion zum Aldehyd sorgen, der dann die eigentliche Wittig-Reaktion eingeht.

Im Vergleich zur sonst in der Terpenchemie üblichen Acetalschutzgruppe für Aldehyde erspart die Acylfunktion eine gesonderte Hydrolyse, da sie im Gegensatz zu Acetalen auch basisch leicht abspaltbar ist. Sie erlaubt aber andererseits die selektive Reaktion nicht geschützter Aldehydfunktionen im Molekül (z. B. (I) Y = CHO) unter nichtprotonischen Bedingungen (z. B. Wittig-Horner-Reaktion, Metallalkyle in nicht proton. Lösungsmitteln) oder unter neutralen Bedingungen (Buddrus-Variante der Wittig-Reaktion).

Die Ausgangsstoffe II und III bzw. IV sind aus leicht zugänglichen Vorprodukten erhältlich. So wird 2-Methyl-4,4-diacyloxy-2-butenal in einfacher Weise nach dem Verfahren der deutschen Patentanmeldung DE-3 125 891 erhalten, auf die hiermit Bezug genommen wird.

Die Komponenten III und IV werden in an sich bekannter Weise aus γ-Chlortiglinaldehyd bzw. γ-Chlortiglinester gewonnen, wobei die Aldehydgruppe z. B. mit Neopentylglykol acetalisiert werden kann.

Die erfindungsgemäßen bifunktionellen $C_{10}$-Bausteine eignen sich hervorragend zur Synthese von Terpenverbindungen, beispielsweise von β-Apo-8'-carotinsäureester oder β-Apo-8'-carotinal durch Umsetzung mit einem Retinylphosphoniumsalz z. B. nach dem Schema

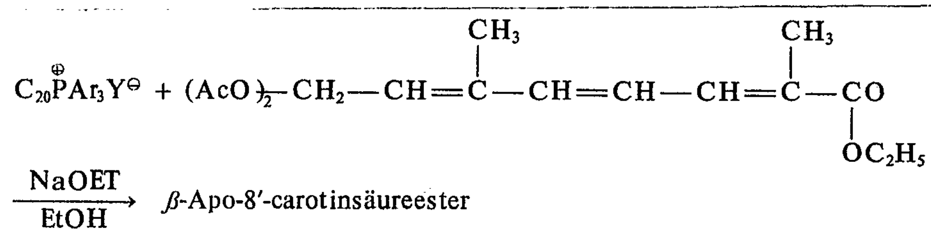


wobei sehr gute Ausbeuten erhalten werden.

In gleicher Weise wird β-Apo-8'-carotinal durch Umsetzung eines Retinylphosphoniumsalzes mit dem cyclischen Acetal des Diacylaldimethyloctatrienals der Formel

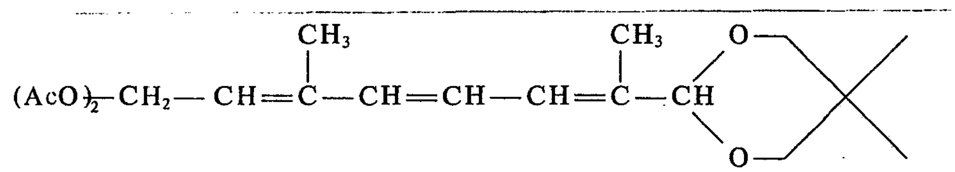

und Hydrolyse des Acetals erhalten.

Gegenüber dem Verfahren der US-Patentschrift 3 989 758 hat die erfindungsgemäße Verwendung

der neuen Zwischenprodukte den Vorteil der erheblich leichteren Zugänglichkeit bei mindestens ebenso guter Eignung für die Herstellung der Endprodukte.

Eine weitere Möglichkeit der Verwendung der neuen $C_{10}$-Bausteine besteht in der Herstellung von $\beta$-Carotin und Retinal. Setzt man Cyclogeranylphosphoniumbromid mit Diacylaldimethyloctatrienal der Formel I (Y=CHO) um, so erhält man Retinalacylal, das mit Natriumethylat in Ethanol in Retinal übergeht.

Wird Retinalacylal weiter umgesetzt mit Retinylphosphoniumsalz, erhält man $\beta$-Carotin.

Alle diese Wittig-Reationen erfolgen unter an sich bekannten Bedingungen wie sie in Org. Reactions, Vol. 14, S. 271—490, beschrieben sind, worauf bezüglich der Einzelheiten der Umsetzung Bezug genommen werden.

## Beispiel 1

### 2,6-Dimethyl-8,8-diacetoxi-2,4,6-octatrien-säureethylester

Zu 500 g Butylenoxid-1,2 wurden unter Rühren bei 30° C 1 Mol (3-Ethoxicarbonyl-3-methyl-2-propenyl)-triphenyl-phosphoniumchlorid und dann allmählich 1 Mol 2-Methyl-4,4-diacetoxi-2-butenal gegeben. Die Reaktionsmischung erwärmte sich während des Zutropfens des Butenals auf ca. 60° C. Man rührte 8 Stunden bei 50° C nach und ließ dann auf Raumtemperatur abkühlen. Die so erhaltene Lösung enthielt neben Triphenylphosphin 0,9 Mol des Octatriensäureesters. Die Lösung kann direkt zu $\beta$-Apocarotinsäureester weiterverarbeitet werden.

Zur Gewinnung des kristallinen Produkts wurde die Reaktionsmischung mit 500 ml Methanol versetzt, auf −10° C abgekühlt und 6 h bei dieser Temperatur gerührt. Danach wurde das ausgefallene Kristallisat abgesaugt und mit Stickstoff trockengeblasen. Man erhielt 220 g Produkt (71%; Reinheit 95%), das nach dem Umkristallisieren aus Ethylacetat bei 106° C schmolz.

$^1$H-NMR: [$\delta$ ppm (Multiplizität, Integral)]:

1,33 (T, 3 H), 2,02 (S, 3 H), 2,04 (S, 3 H), 2,11 (S, 6 H), 4,25 (Q, 2 H), 5,67 (D, 1 H), 6,57 (DD, 1 H), 6,66 (D, 1 H), 7,25 (D, 1 H), 7,57 (D, 1 H).

## Beispiel 2

### 2,6-Dimethyl-8,8-diacetoxid-2,4,6-octatriensäureethylester

42,5 g (0,1 Mol) (3-Ethoxicarbonyl-3-methyl-2-propenyl)-triphenyl-phosphoniumchlorid wurden in 100 ml trockenem Dioxan vorgelegt. Bei 10° C wurden unter Rühren in einer $N_2$-Atmosphäre zunächst 47 g (0,11 Mol) n-Butyllithium (15% in Hexan) zugetropft; man rührte 10 Minuten nach und tropfte dann 20 g (0,1 Mol) 2-Methyl-4,4-diacetoxi-2-butenal zu. Schließlich rührte man unter allmählichem Erwärmen auf Raumtemperatur 8 Stunden nach.

Zur Aufarbeitung wurden unter Eiskühlung 100 ml Hexan und 100 ml $MeOH/H_2O$ 6 : 4 zugegeben. Nach Trennung der Phasen wurde die Hexanphase noch zweimal mit je 100 ml wäßrigem Methanol (60%) gewaschen, mit $Na_2SO_4$ getrocknet und im Rotationsverdampfer bei 40° C und Wasserstrahlvakuum eingeengt. Als Rückstand verblieben 40,9 g Kristallisat, das nach NMR zu 65% aus 2,6-Dimethyl-8,8-diacetoxid-2,4,6-octatriensäureethylester und zu 35% aus Triphenylphosphinoxid bestand. Durch Aufrühren dieses Kristallisats in 50 ml Methanol bei 0° C erhielt man nach Filtration und Trocknen ($Na_2SO_4$) 20,5 g des Produkts in über 90%iger Reinheit.

## Beispiel 3

### 2,6-Dimethyl-8,8-diacetoxi-2,4,6-octatriensäureethylester

Zu 100 ml trockenem Tetrahydrofuran wurden unter Stickstoff 0,96 g (40 mMol) Natriumhydrid gegeben. Dazu ließ man bei 50° C unter Rühren 4,66 g (20 mMol) (3-Ethoxicarbonyl-3-methyl-2-propenyl)-phosphonester und dann 4,0 g (20 mMol) 2-Methyl-4,4-diacetoxi-2-butenal zutropfen. Man rührte 6 Stunden bei 50° C nach, ließt auf Raumtemperatur abkühlen, tropfte 50 ml $H_2O$ zu, trennte die Phasen und extrahiert die wäßrige Phase noch dreimal mit je 50 ml Diethylether und die vereinigten organischen Phasen schließlich noch zweimal mit je 100 ml gesättigter NaCl-Lösung.

Nach dem Trocknen ($Na_2SO_4$) und Einengen blieb ein teilkristalliner Rückstand (5,71 g), der nach NMR ca. 65% 2,6-Dimethyl-8,8-diacetoxi-2,4,6-octatriensäureethylester enthielt. Durch Kristallisation aus Ethylacetat erhielt man 3,2 g reines Produkt.

## Beispiel 4

### 2,6-Dimethyl-8,8-diacetoxi-2,4,6-octatrienal-neopentylacetal

46 g (0,1 Mol) 3-Methyl-3-(5,5-dimethyl-1,3-dioxolan-2-yl)-2-propenyltriphenylphosphoniumchlorid wurden in 100 g Butylenoxid-1,2 suspendiert. Unter Rühren bei Raumtemperatur wurden 20 g (0,1 Mol) 2-Methyl-4,4-diacetoxi-2-butenal zugetropft. Nach 30 h Rühren bei Raumtemperatur war die Umsetzung gemäß dünnschichtchromatographischer Kontrolle (Kieselgel, Laufmittel Diisopropylether/n-Hexan 1 : 1) vollständig. Das entstandene Triphenylphosphinoxid wurde durch Filtration des Ansatzes über eine kurze Kieselgelsäure (100 g Kieselgel, Nachwaschen mit $Et_2O$) abgetrennt. Nach dem Einengen unter vermindertem Druck erhielt man 32 g öliges 2,6-Dimethyl-8,8-diacetoxi-2,4,6-octatrienal-neopentylacetal als Isomerengemisch. Das all-E-Isomere ist in diesem Gemisch zu etwa 80% enthalten und wurde für spektroskopische Zwecke durch präp. Hochdruckflüssigchromatographie, 150 x 10 Si5, Laufmittel Cyclohexan/Diethylether 10 : 1,5, Detektion: UV = 280 nm) abgetrennt. Es resultierte ein farbloses Öl.

$^1$H-NMR [$\delta$ ppm (Multiplizität, Integral)]:
0,76 (S, 3 H), 1,24 (S, 3 H), 1,88 (S, 3 H), 1,99 (S, 3 H), 2,08—2,09 (4 S, 6 H), 3,52 (4 D, 2 H), 3,68 (4 D, 2 H), 4,77 (S, 1 H), 5,54 (D, 1 H), 6,2—6,4 (M, 2 H), 6,62 (D, 1 H), 7,5 (D, 1 H).

## Beispiel 5

### 2,6-Dimethyl-8,8-diacetoxi-2,4,6-octatrienal

34,5 g (0,1 Mol) 3-Formyl-buten-2-yl-triphenylphosphin (hergestellt nach den Angaben der Belg. PS 656 433) und 20,0 g (0,1 Mol) 2-Methyl-4,4-diacetoxi-2-butenal wurden in 100 ml Methyl-t-butylether vorgelegt. Unter Rühren wurde 5 Stunden auf 60° C erwärmt, danach war dünnschichtchromatographisch (n-Hexan/Ether 6 : 4) kein Edukt mehr nachweisbar. Nach Abkühlen der Reaktionsmischung auf Raumtemperatur wurde weiter auf −20° C gekühlt und die Mischung 2 Stunden bei dieser Temperatur gerührt; dabei fiel ein Kristallisat an, das sich nach der Isolierung als reines Triphenylphosphinoxid erwies. Die Mutterlauge wurde im Rotationsverdampfer eingeengt. Es verblieben 28,6 g hellgelbes Öl, das nach NMR-spektroskopischer Kontrolle 84,5% des Octatrienals als Isomerengemisch und 15,5% Triphenylphosphinoxid enthielt. Für weitere Umsetzungen kann das so erhaltene Produkt ohne weitere Reinigung eingesetzt werden. Für spektroskopische Zwecke wurden aus dem Rohprodukt durch Kristallisation aus n-Hexan/Diethylether 1 : 2 reines all-E-2,6-Dimethyl-8,8-diacetoxi-2,4,6-octatrienal als weiße bis schwach gelbliche perlmuttartige glänzende Plättchen isoliert. Schmelzpunkt: 99,5° C.

$^1$H-NMR [$\delta$ ppm (Multiplizität, Integral)]:
1,91 (S, 3 H), 2,06 (S, 3 H), 2,10 (S, 6 H), 5,76 (D, 1 H), 6,67 (D, 1 H), 6,82 (DD, 1 H), 6,94 (D, 1 H), 7,58 (D, 1 H), 9,50 (S, 1 H).

## Beispiel 6

### $\beta$-Apo-8'-carotinsäureester

In 2000 ml Ethanol wurden 660 g (1,05 Mol) Retinyl-triphenylphosphoniumhydrogensulfat und 310 g (1 Mol) 2,6-Dimethyl-8,8-diacetoxi-2,4,6-octatriensäureethylester (rein) vorgelegt. Unter Rühren und Wasserkühlung tropfte man 2000 ml 2 N Natriumethanolat in Ethanol so zu, daß die Temperatur der Reaktionsmischung nicht über 30° C stieg. Man rührte 24 Stunden bei 30° C nach und arbeitete dann bei 55—60° C auf, indem zur Reaktionsmischung 2000 ml n-Heptan und 2700 ml $H_2O$ gegeben wurden, die wäßrige Unterphase abgetrennt und die organische Phase noch dreimal mit je 2000 ml wäßrigem Methanol (60%) gewaschen wurde. Danach konzentrierte man unter Wasserauskreisen auf 900 ml auf, gab 560 ml Methanol und 300 ml Isopropanol zu und kühlte allmählich auf 0° C ab. Nach dem 2stündigen Rühren bei 0° C filtrierte man ab, wusch zweimal mit je 300 ml eiskaltem Methanol nach und blies mit Stickstoff trocken. Ausbeute 236,5 g Schmelzpunkt: 137—138° C (keine Depression mit authentischem Muster). Spez. Extinktion: $E_1^1$ = 2550 (bei 449 nm in Cyclohexan).

Die Mutterlauge enthielt ein Gemisch stereoisomerer $\beta$-Apo-8'-carotinsäureester, in dem das all-E-Isomere zu 18% enthalten ist. Durch photochemische Isomerisierung in Gegenwart von Bengalrosa als Sensibilisator kann der all-E-Anteil innerhalb von 10 Minuten Bestrahlungsdauer auf 74,5% gesteigert werden. Nach Kristallisation bei 0° C wie oben erhielt man weitere 145,2 g reinen $\beta$-Apo-8'-carotinsäureester.

Schmelzpunkt: 137—138° C (keine Depression mit authent. Muster)
Spez. Extinktion: $E_1^1$ = 2562 (bei 449 nm in Cyclohexan)

## Beispiel 7

### β-Apo-8'-carotinsäureester

Man legte 600 g einer nach Beispiel 1 erhaltenen Reaktionsmischung vor, die nach NMR 0,5 Mol 2,6-Dimethyl-8,8-diacet-oxi-2,4,6-octatriensäureethylester enthielt, sowie 800 ml Ethanol und 330 g (0,525 Mol) Retinyl-triphenyl-phosphoniumhydrogensulfat. Unter Wasserkühlung wurden 1250 ml 2 N Natriummethanolat in Ethanol so zugetropft, daß die Temperatur 30° C nicht überstieg. Man rührte 30 Stunden bei 30° C nach und arbeitete bei 55—60° C wie in Beispiel 6 angegeben auf.

Erstkristallisat: 98,3 g
Kristallisat nach Isomerisierung: 59,5 g
Die beiden Kristallisate wurden vereinigt und aus 2000 ml Ethanol/n-Heptan 4 : 1 umkristallisiert.
Ausbeute: 148,6 g (64,6%)
Schmelzpunkt: 136—138° C
Spez. Extinktion: 2548 (in Cyclohexan bei 449 nm)

## Beispiel 8

### β-Apo-8'-carotinsäureethylester

Zu 1000 ml 1molarer Retinyl-triphenyl-phosphoniumhydrogensulfatlösung (hergestellt nach den Angaben der DAS 2 729 974) wurde eine Lösung von 310 g (1 Mol) 2,6-Dimethyl-8,8-di-acetoxi-2,4,6-octatriensäureethylester und 290 g (5 Mol) Magnesiumhydroxid gegeben. Die Suspension wurde 20 h bei 40° C gerührt. Danach wurde analog Beispiel 1 aufgearbeitet.

Erstkristallisat: 224,6 g
Schmelzpunkt: 136—137,5° C
Spez. Extinktion: 2555 (bei 449 nm in Cyclohexan)
Kristallisat nach Isomerisierung: 94,8 g
Schmelzpunkt: 136—138° C
Spez. Extinktion: 2550 (bei 449 nm in Cyclohexan)

## Beispiel 9

### β-Apo-8'-carotinal

Zu einer Lösung von 57 g (0,1 Mol) Retinyl-triphenyl-phosphoniumchlorid in 300 ml Methanol wurden bei 0° C unter Rühren gleichzeitig 200 ml 2 N Natriummethanolat (in Methanol) und eine Lösung von 35,2 g (0,1 Mol) 2,6-Dimethyl-8,8-di-acetoxi-2,4,6-octatrienal in 50 ml Methanol zugetropft. Nach beendetem Zutropfen wurde 2 h bei 0° C und dann 2 h bei Raumtemperatur nachgerührt. Dann gab man bei 40° C 500 ml n-Heptan und 500 ml $H_2O$ zu und trennte die Phasen. Darauf wurde die org. Phase noch zweimal mit je 300 ml 60%igem wäßrigem Methanol gewaschen und schließlich unter Rühren mit 100 ml 3 N $H_2SO_4$ bei 40° C behandelt. Nach 2 Stunden ließ man auf Raumtemperatur abkühlen, rührte noch 5 Stunden nach und filtrierte ab. Die Rohausbeute betrug 32,3 g. Nach Umkristallisieren (Lösen in 65 ml $CHCl_3$, Zugabe von 160 ml Ethanol/Heptan, Abkühlen auf 0° C) erhielt man 28,1 g reines β-Apo-8'-carotinal.

Schmelzpunkt: 138—140° C
Spez. Extinktion: 2640 (bei 457 nm in Cyclohexan)

## Beispiel 10

### Retinal

47,9 g (0,1 Mol) Cyclogeranyl-triphenyl-phosphoniumbromid wurden in 500 ml Dioxan (dest. über KOH) vorgelegt. Man tropfte unter einer $N_2$-Atmosphäre unter Rühren bei 10° C 64 g (0,15 Mol) Butyllithium (15% in Hexan) innerhalb 15 Minuten zu und rührte die Mischung 2 Stunden bei 10° C nach. Danach tropfte man unter den gleichen Bedingungen 31,5 g (0,1 Mol, 84,5%ig, vgl. Beispiel 5) 2,6-Dimethyl-8,8-diacetoxi-2,4,6-octatrienal gelöst in 50 ml Dioxan zu, und rührte anschließend die Mischung unter Erwärmen auf Raumtemperatur 2 Stunden und bei 40° C 3 Stunden nach. Danach kühlte man auf 10° C ab und tropfte zunächst 20 ml Dioxan/Wasser 1 : 1, dann 200 ml Wasser und schließlich 200 ml

Diisopropylether zu. Die Dioxan/Wasser-Phase wurde abgetrennt und zweimal mit je 200 ml Diisopropylether extrahiert. Die vereinigten organischen Phasen wurden getrocknet ($Na_2SO_4$), filtriert und auf 100 ml eingeengt. Unter Rühren bei Raumtemperatur wurde eine Lösung von 0,2 Mol Natriumethanolat in 100 ml Methanol zugetropft und 4 Stunden nachgerührt. Dann wurden 100 ml n-Heptan und 100 ml Wasser zugegeben, die Phasen wurden getrennt, die wäßrig-methanolische noch 2 × mit je 100 ml n-Heptan extrahiert, die vereinigten organischen Phasen auf 60 ml konzentriert und bei −20° C kristallisiert. Das Kristallisat wurde über eine gekühlte Fritte abgesaugt und mit $N_2$ trockengeblasen.

Ausbeute: 20,5 g
Schmelzpunkt: 61,5 bis 62,5° C
Spez. Extinktion: 1530 (bei 381 nm in Ethanol)

**Patentansprüche**

1. Verbindungen der Formel

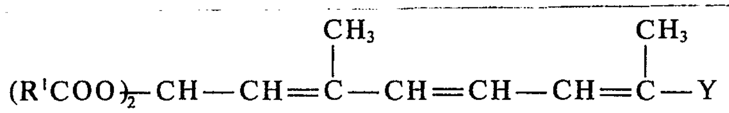

in der Y für den Rest $-COOR^1$, eine Formylgruppe oder eine acetalisierte Formylgruppe steht, wobei $R^1$ einen niedrigmolekularen Alkylrest bedeutet.

2. Verbindungen gemäß Anspruch 1, dadurch gekennzeichnet, daß Y die Reste $-COOR^1$, $-CHO$ oder

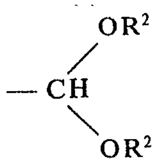

bedeutet, in denen $R^1$ einen Alkylrest mit 1 bis 5 Kohlenstoffatomen und $R^2$ einen Alkylrest mit 1 bis 5 Kohlenstoffatomen bedeutet, wobei die zwei $R^2$-Reste zusammen einen Alkylenrest oder einen Alkenylenrest mit 2 bis 4 Kohlenstoffatomen in der Kette bedeuten können.

3. Verbindung gemäß Anspruch 1, dadurch gekennzeichnet, daß Y den Rest $-COOC_2H_5$ und $R^1$ Methyl bedeutet.

4. Verbindung gemäß Anspruch 1, dadurch gekennzeichnet, daß Y den Rest $-CHO$ und $R^1$ Methyl bedeutet.

5. Verbindung gemäß Anspruch 1, dadurch gekennzeichnet, daß Y den Rest

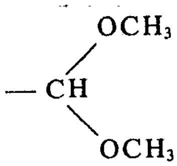

und $R^1$ Methyl bedeutet.

6. Verbindung gemäß Anspruch 1, dadurch gekennzeichnet, daß Y den Rest

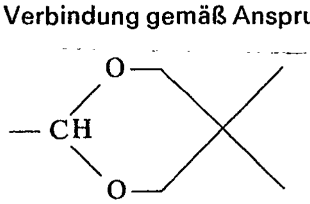

und $R^1$ Methyl bedeutet.

7. Verfahren zur Herstellung von Verbindungen gemäß Anspruch 1, dadurch gekennzeichnet, daß man ein Acylal des Methylbutendials der Formel II

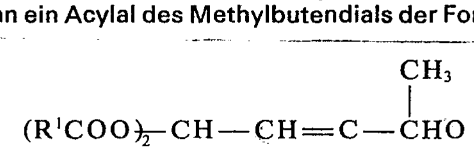

(II)

mit einem Triarylphosphoniumsalz der Formel III

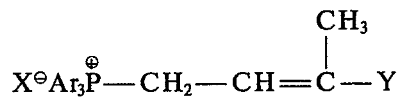 (III)

in Gegenwart eines Epoxids oder unter nicht protonischen basischen Bedingungen umsetzt, wobei $R^1$ und Y die in Anspruch 1 angegebenen Bedeutungen haben, Ar einen Arylrest und X Chlor oder Brom bedeutet.

8. Verfahren zur Herstellung von Verbindungen gemäß Anspruch 1, dadurch gekennzeichnet, daß man ein Acylal des Methylbutendials der Formel II

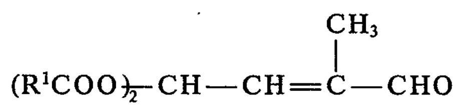

mit einem Phosphonat der Formel IV

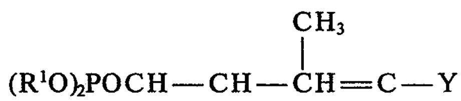

umsetzt, wobei $R^1$ und Y die in Anspruch 1 angegebenen Bedeutungen haben.

9. Verwendung von Verbindungen gemäß Anspruch 1 zur Herstellung von Terpenverbindungen.

10. Verwendung gemäß Anspruch 9 von Verbindungen gemäß Anspruch 1 zur Herstellung von β-Apo-8'-carotinsäureester, β-Apo-8'-carotinal, Retinal oder β-Carotin.

**Claims**

1. A compound of the formula

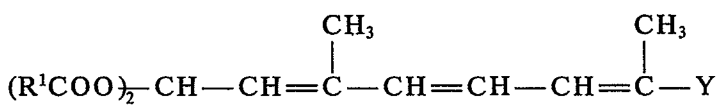

where Y is $-COOR^1$, formyl or an acetalated formyl group and $R^1$ is a low molecular weight alkyl radical.

2. A compound as claimed in claim 1, wherein Y ist $-COOR^1$, $-CHO$ or

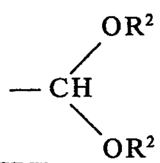

where $R^1$ and $R^2$ are each alkyl of 1 to 5 carbon atoms, and the two $R^2$ radicals together can form an alkylene radical or an alkenylene radical of 2 to 4 chain carbon atoms.

3. A compound as claimed in claim 1, wherein Y is $-COOC_2H_5$ and $R^1$ ist methyl.

4. A compound as claimed in claim 1, wherein Y is $-CHO$ and $R^1$ is methyl.

5. A compound as claimed in claim 1, wherein Y is

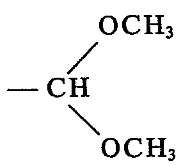

and $R^1$ is methyl.

6. A compound as claimed in claim 1, wherein Y is

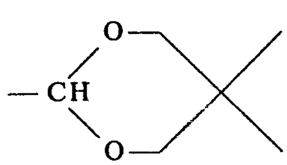

and $R^1$ is methyl.

7. A process for the preparation of a compound as claimed in claim 1, wherein an acylal of methylbutenedial of the formula II

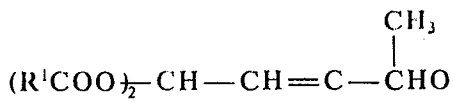

(II)

is reacted with a triarylphosphonium salt of the formula III

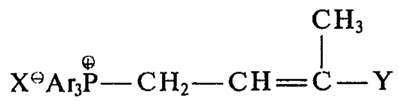

(III)

where $R^1$ and Y have the meanings given in claim 1, Ar is aryl and X is chlorine or bromine, in the presence of an epoxide or under non-protic basic conditions.

8. A process for the preparation of a compound as claimed in claim 1, wherein an acylal of methylbutenedial of the formula II

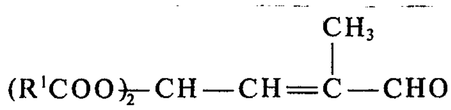

is reacted with a phosphonate of the formula IV

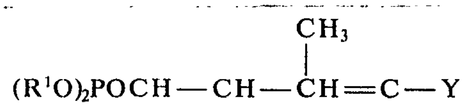

where $R^1$ and Y have the meanings given in claim 1.

9. The use of a compound as claimed in claim 1 for the production of terpene compounds.

10. The use, as claimed in claim 9, of a compound as claimed in claim 1 for the production of $\beta$-apo-8'-carotenic acid ester, $\beta$-apo-8'-carotenal, retinal or $\beta$-carotene.

**Revendications**

1. Composés de formule

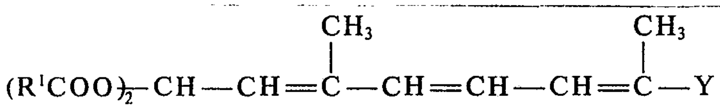

dans laquelle Y est mis pour le radical $-COOR^1$, un groupe formyle ou un groupe formyle acétalisé, $R^1$ représentant un radical alkyle inférieur.

2. Composés selon la revendication 1, caractérisés en ce que Y représente les radicaux $-COOR^1$, $-CHO$ ou

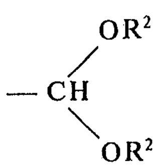

dans lesquels $R^1$ représente un radical alkyle à 1—5 atomes de carbone et $R^2$ un radical alkyle à 1—5 atomes de carbone, les deux radicaux $R^2$ pouvant représenter ensemble un radical alkylène ou un radical alcényle à 2—4 atomes de carbone dans la chaîne.

3. Composé selon la revendication 1, caractérisé en ce que Y représente le radical $-COOC_2H_5$ et $R^1$ le radical méthyle.

4. Composé selon la revendication 1, caractérisé en ce que Y représente le radical $-CHO$ et $R^1$ le radical méthyle.

5. Composé selon la revendication 1, caractérisé en ce que Y représente le radical

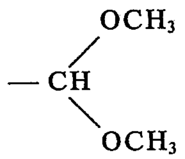

et $R^1$ le radical méthyle.

6. Composé selon la revendication 1, caractérisé en ce que Y représente le radical

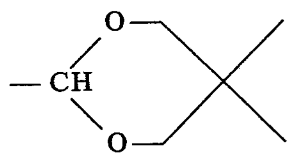

et $R^1$ le radical méthyle.

7. Procédé pour la préparation de composés selon la revendication 1, caractérisé en ce qu'on fait réagir un acylal du méthylbutène-dial de formule II

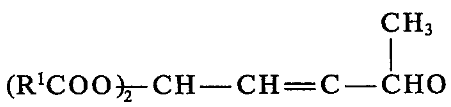

(II)

avec un sel de triarylphosphonium de formule III

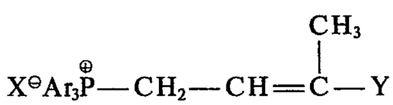

(III)

en présence d'un époxyde ou dans des conditions basiques non protoniques, $R^1$ et Y ayant les significations données dans la revendication 1, Ar représentant un radical aryle et X un atome de chlore ou de brome.

8. Procédé pour la préparation de composés selon la revendication 1, caractérisé en ce qu'on fait réagir un acylal du méthylbutène-dial de formule II

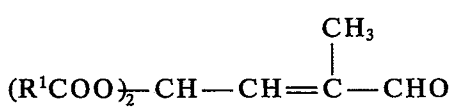

(II)

avec un phosphonate de formule IV

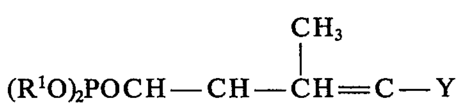

(IV)

$R^1$ et Y ayant les significations données dans la revendication 1.

9. Utilisation de composés selon la revendication 1 pour la préparation de composés terpéniques.

10. Utilisation selon la revendication 9 de composés selon la revendication 1 pour la préparation d'esters d'acide $\beta$-apo-8'-caroténique, de $\beta$-apo-8'-caroténal, de rétinal ou de $\beta$-carotène.